# EUROPEAN PATENT APPLICATION

(11) **EP 2 796 548 A1**
(43) Date of publication of application: **29.10.2014**
(21) Application number: 13165298.4
(22) Date of filing: 25.04.2013
(51) Int. Cl.: C12N 9/04, C12N 15/62, C12P 17/18

(54) **Stereoselective production of (R)-3-quinuclidinol**

(71) Applicant: Interquim, S.A., 08173 Sant Cugat del Vallès (ES)
(72) Inventor: Pellicer Moya, María Teresa, 08173 SANT CUGAT DEL VALLÈS (ES); Marquillas Olondriz, Francisco, 08173 SANT CUGAT DEL VALLÈS (ES); Planas Sauter, Antoni, 08017 BARCELONA (ES); Perez Javierre, Francesc Xavier, 08230 Matadepera (ES)
(74) Representative: ZBM Patents - Zea, Barlocci & Markvardsen

(57) **Abstract**

The present invention refers to an isolated polynucleotide sequence encoding a chimeric fusion protein comprising a glucose 1-dehydrogenase amino acid sequence or a functionally equivalent variant thereof and a NADPH-dependent 3-quinuclidinone reductase amino acid sequence or a functionally equivalent variant thereof. By expressing said fusion protein in microbial cells, 3-quinuclidinone is stereoselectively reduced to (R)-3-quinuclidinol in a very efficient manner.

## Description

### BACKGROUND OF THE INVENTION

Chemical processes for obtaining optically pure compounds include enantiomer separation from a racemic mixture, derivation from natural substances and asymmetric synthesis.

Biocatalytic transformation using enzymes or microorganisms has been applied to the asymmetric reduction of ketones. These biocatalytic processes are more environmentally sustainable and thus more attractive for pharmaceutical manufacturing. In recent years, the asymmetric reduction of ketones with biocatalysts has been reported for the production of chiral alcohols and applied to industry.

(R)-3-quinuclidinol is a common building block of a number or active pharmaceutical ingredients currently in the market, such as aclidinium and solifenacin, and in phase III clinical trials. For the stereospecific production of (R)-3-quinuclidinol, chemical and biochemical methods have been developed, most of them involving the resolution of a racemic mixture of 3-quinuclidinol with a maximal theoretical yield of 50%.

A number of enzymes have been reported as catalysts for the bioreduction of 3-quinuclidinone to (R)-3-quinuclidinol ((*R*)-(-)-3-quinuclidinol). Uzura et al (Appl. Microbiol Biotechnol. (2009) 83: 617-626) described a stereoselective biocatalytical method to produce (R)-3-quinuclidinol with >99.9% enantiomeric excess within 21 hours of reaction. *Escherichia coli* cells co-expressing a protein with 3-quinuclidinone reductase activity and a cofactor regeneration system in two independent plasmids were able to produce (R)-3-quinuclidinol. More recently, Isotani et al (Int. J. Mol. Sci. (2012) 13, 13542-13553) described a bioreduction system for synthesizing (*R*)-3-quinuclidinol using a recombinant *Escherichia coli* cell biocatalyst possessing a 3-quinuclidinone reductase from *Microbacterium luteolum* and the *Leifsonia sp.* alcohol dehydrogenase (LSADH). Both genes were cloned in a single construct under the control of independent promoters. The resulting *E. coli* biocatalyst exhibited a level of production of (*R*)-3-quinuclidinol of 150 mg/mL and high enantioselectivity.

However, there is still a need for more efficient processes for the production of (*R*)-3-quinuclidinol which can be utilized in the industrial production of this alcohol.

### SUMMARY OF THE INVENTION

The authors of the present invention have found that by expressing a fusion protein comprising a glucose 1-dehydrogenase (GDH) and a 3-quinuclidinone reductase (Qred) under the control of a single promoter in microbial cells, 3-quinuclidinone is stereoselectively reduced to (R)-3-quinuclidinol in a very efficient manner. Indeed, it has been shown (see Examples below) that expression of Qred is significantly increased when expressed as a chimeric protein GDH-Qred. Although the authors of the present invention do not wish to be bound by any theory, it seems that GDH would act as a carrier pulling Qred expression, which in another way has been shown to be very low in bacterial cells. Moreover, at the catalytic level, cofactor (NADP⁺) regeneration is equimolar to the reductase activity. Equimolarity and the spatial nearness of both enzymes thus enhance the velocity and efficiency of the enzymatic catalysis.

As it is shown in the Examples accompanying the present invention, the expression of a gene construct coding for both, GDH and Qred proteins, under the control of a single promoter in microbial cells produces a fusion protein with both high dehydrogenase and high reductase activity. Whole cells, processed cells or immobilized forms thereof expressing said fusion protein are capable of stereoselectively reducing 3-quinuclidinone to (R)-3-quinuclidinol in a very efficient manner.

Therefore, in a first aspect, the present invention refers to an isolated polynucleotide sequence encoding a chimeric fusion protein comprising a glucose 1-dehydrogenase amino acid sequence or a functionally equivalent variant thereof and a NADPH-dependent 3-quinuclidinone reductase amino acid sequence or a functionally equivalent variant thereof.

In a second aspect, the invention refers to a genetic construct comprising a polynucleotide according to the invention.

In a third aspect, the invention relates to a fusion protein encoded by a polynucleotide sequence or a genetic construct according to the invention.

In a further aspect, the invention refers to a host cell comprising a polynucleotide sequence or a genetic construct or a fusion protein according to the invention, wherein said polynucleotide sequence is expressible in said host cell.

In another aspect, the invention refers to the use of a polynucleotide or a genetic construct or a fusion protein or a host cell according to the invention for the manufacture of (R)-3-quinuclidinol.

In a further aspect, the invention relates to a method for the manufacture of (R)-3-quinuclidinol comprising
i) bringing into contact 3-quinuclidinone and glucose with a host cell according to the invention; and
ii) recovering the (R)-3-quinuclidinol so produced.

### DETAILED DESCRIPTION OF THE INVENTION

As mentioned above, the authors of the present invention have found that by expressing a fusion protein comprising a glucose 1-dehydrogenase amino acid sequence and a 3-quinuclidinone reductase amino acid sequence in microbial cells, 3-quinuclidinone is stereoselectively reduced to (R)-3-quinuclidinol in a very efficient manner.

Therefore, in a first aspect, the present invention refers to a polynucleotide sequence encoding a chimeric fusion protein comprising a glucose 1-dehydrogenase amino acid sequence or a functionally equivalent variant thereof and a NADPH-dependent 3-quinuclidinone reductase amino acid sequence or a functionally equivalent variant thereof.

As used herein, "a" or "an" means "at least one" or "one or more" unless otherwise indicated.

The term "polynucleotide" is used herein interchangeably with "nucleotide sequence" and relates to any polymeric form of nucleotides of any length and composed of ribonucleotides and/or deoxyribonucleotides. The term includes both single-stranded and double-stranded polynucleotides as well as modified polynucleotides (methylated, protected and the like).

Techniques for the manipulation of nucleic acids, such as, e.g., subcloning, labeling probes (e.g., random-primer labeling using Klenow polymerase, nick translation, amplification), sequencing, hybridization and the like are well described in the scientific and patent literature.

3-quinuclidinone reductase (Qred) is a nicotinamide adenine dinucleotide phosphate (NADPH)-dependent carbonyl reductase which catalyzes the stereospecific reduction of 3-quinuclidinone to (R)-3-quinuclidinol. Thus, Qred requires NADPH as cofactor for the reaction. The glucose 1-dehydrogenase (GDH) enzyme acts as a cofactor regeneration enzyme since it catalyzes the chemical reaction of beta-D-glucose + NADP to yield D-glucono-1,5-lactone + NADPH.

In a preferred embodiment of the invention, said glucose 1-dehydrogenase sequence comprises *Bacillus megaterium* glucose 1-dehydrogenase sequence (GenBank Accession No: YP_003563827, Version 1 from 23-December-2012). In a particular embodiment, said sequence comprises SEQ ID NO: 1. In another particular embodiment, said sequence consists in SEQ ID NO: 1.

Qred enzymes which can be used according to the present invention include, for example, the NADH-dependent 3-Quinuclidinone Reductase sequence (QNR or bacC) from *Microbacterium luteolum* (Isotani et al. Appl Environ Microbiol. 2013 Feb; 79(4):1378-84), *Rhodotorula mucilaginosa* NADPH-dependent 3-quinuclidinone reductase sequence (GenBank Accession No. BAH28833, Version 1 from 11-June-2009) or a *Rhodotorula rubra* NADPH-dependent 3-quinuclidinone reductase sequence. It is understood by the skilled person in the art that *Rhodotorula mucilaginosa* and *Rhodotorula rubra* refers to the same microorganism. Therefore, the terms *Rhodotorula mucilaginosa* and *Rhodotorula rubra* are used herein interchangeably. In a preferred embodiment, said NADPH-dependent 3-quinuclidinone reductase sequence comprises *Rhodotorula rubra* NADPH-dependent 3-quinuclidinone reductase sequence which is also described in document JP2007124922. In a particular embodiment, said sequence comprises SEQ ID NO: 2. In another particular embodiment, said sequence consists in SEQ ID NO: 2.

The invention should be construed to include DNA encoding functional equivalent variants of said enzymes. The term "functional equivalent variant", as used herein relates to any polypeptide which sequence can be obtained from the sequence of Qred or GDH as defined above by means of insertion of one or more nucleotides in the sequence, the addition of one or more nucleotides in any end or inside the sequence, or the deletion of one or more nucleotides in any end or inside the sequence and which substantially preserves the biological activity of said enzymes.

Variants of said enzymes may be obtained by substituting nucleotides within the polynucleotide accounting for codon preference in the host cell that is to be used to produce the enzyme. Such "codon optimization" can be determined via computer algorithms which incorporate codon frequency tables such as "Ecohigh. Cod" for codon preference of highly expressed bacterial genes as provided by the University of Wisconsin Package Version 9.0, Genetics Computer Group, Madison,Wis. Other useful codon frequency tables include "Celegans_ high.cod", "Celegans _low.cod", Drosophila_high.cod", "Human_ high.cod", "Maize_ high.cod", and "Yeast_ high.cod".

Variants of Qred or GDH may be generated by making conservative amino acid changes and testing the resulting variant in functional assays known in the art. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine.

The functionally equivalent variants of Qred or GDH include polypeptides which are substantially homologous to the native enzyme. The expression "substantially homologous", as used herein, relates to any of the nucleotide sequences described above when its nucleotide sequence has a degree of identity with respect to the nucleotide sequence of the invention of at least 60 percent, advantageously of at least 70 percent, preferably of at least 85 percent, and more preferably of at least 95 percent. A nucleotide sequence that is substantially homologous to the nucleotide sequence of the invention can typically be isolated from a producer organism of the polypeptide of the invention based on the information contained in said nucleotide sequence, or it is constructed based on the DNA sequence described above. The degree of identity between two polynucleotides is determined using computer algorithms and methods that are widely known for the persons skilled in the art. The identity between two amino acid sequences is preferably determined by using the BLASTN algorithm [BLAST Manual, Altschul, S., et al., NCBI NLM NIH Bethesda, Md. 20894, Altschul, S., et al., J. Mol. Biol. 215: 403-410 (1990)]. BLAST and BLAST 2.0 are used, with the parameters described herein, to determine percent sequence identity. Software for performing BLAST analyses is publicly available through the National Center for Biotechnology Information. This algorithm involves first identifying high scoring sequence pairs (HSPs) by identifying short words of length W in the query sequence, which either match or satisfy some positive- valued threshold score T when aligned with a word of the same length in a database sequence. T is referred to as the neighborhood word score threshold (Altschul et al, supra). These initial neighbourhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always 0) and N (penalty score for mismatching residues; always 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sd. USA, 1989, 89:10915) alignments (B) of 50, expectation (E) of 10, M=5, N=-4, and a comparison of both strands.

As those skilled in the art will appreciate, variants or fragments of Qred and GDH can be generated using conventional techniques, such as mutagenesis, including creating discrete point mutation(s), or by truncation. For instance, mutation can give rise to variants which retain substantially the same, or merely a subset, of the biological activity of a polypeptide from which it was derived.

The terms "fusion protein" and "chimeric protein" are herein indistinctively used and refer to a protein created through the joining of two or more genes which originally code for separate proteins.

The construction of a fusion protein involves the linking of two macromolecules by a linker sequence. The macromolecules involved usually involve proteins and globular domains of proteins. According to the present invention a linker (or "spacer") peptide is placed in between both proteins. According to the invention, said linker amino acid sequence acts as a hinge region between said proteins or domains, allowing them to move independently from one another while maintaining the three-dimensional form of the individual domains but keeping them in close proximity in space. In this sense, a possible intermediate amino acid sequence according to the invention would be a hinge region characterized by a structural ductility allowing this movement.

Linkers can be classified into three categories according to their structures: flexible linkers, rigid linkers, and *in vivo* cleavable linkers. Besides the basic role in linking the functional domains together (as in flexible and rigid linkers) or releasing the free functional domain *in vivo* (as in *in vivo* cleavable linkers), linkers may offer many other advantages for the production of fusion proteins, such as improving biological activity, increasing expression yield, and achieving desirable pharmacokinetic profiles. Also, linkers enable protein purification. Linkers in protein or peptide fusions can be engineered with cleavage sites for proteases or chemical agents which enable the liberation of the two separate proteins. This technique is often used for identification and purification of proteins, by fusing as "tag" for example, a GST protein, a FLAG peptide, a hexa-his peptide (6xHis-tag) which can be isolated using known techniques in the state of the art, such as affinity chromatography. Fusion proteins can also be manufactured with toxins or antibodies attached to them in order to study disease development. Stable peptide sequences that are used as linkers include, for example, the glycine-serine linker (GGGGS)n (SEQ ID NO: 12) and α-helix-forming peptide linkers, such as A(EAAAK)nA (SEQ ID NO: 13) (n = 2-5) (Arai R. et al. PEDS. Volume 14, Issue 8P, 529-532). Sequences made up of Ala and Pro have also been described. Indeed, by varying the number of Ala-Pro pairs the flexibility of the linker can be modulated. In a particular embodiment, said intermediate amino acid sequence is a flexible linker. The effect of the linker region is to provide space between the Qred and the GDH domains. It is thus assured that the secondary structure of the Qred is not affected by the presence of the GDH and vice versa. The spacer is preferably of a polypeptide nature.

The linker peptide preferably comprises at least 2 amino acids, at least 3 amino acids, at least 5 amino acids, at least 10 amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids or approximately 100 amino acids.

In a particular embodiment, a preferred linker according to the present invention comprises a peptide sequence with a length of 5 to 25 amino acids. In a more preferred embodiment said sequence is a peptide sequence of 15 amino acids. In a more particular embodiment, said linker sequence is a sequence made up of Thr and Pro pairs. More particularly, said sequence is SEQ ID NO: 3.

In a particular embodiment of the invention, the polynucleotide of the invention may be contained in a genetic or DNA construct. Thus, in another aspect, the invention relates to a DNA construct comprising a polynucleotide sequence according to the invention, wherein said polynucleotide sequence is operatively bound to a promoter sequence. Concerning nucleic acids or polynucleotides, "operatively bound" means that one nucleotide region is put in functional relationship with another nucleotide sequence.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter, including e.g. attenuators or enhancers, but also silencers. A "constitutive" promoter is a promoter that is active in most tissues under most physiological and developmental conditions. An "inducible" promoter is a promoter that is physiologically or developmentally regulated, e.g. by the application of a chemical inducer.

The polynucleotide or construct of the invention may be inserted in a suitable expression vector. Thus, an additional object of the present invention is an expression vector that comprises said polynucleotide or DNA construct. The choice of expression vector in the different embodiments of the invention will depend on the host cell wherein said expression vector is going to be inserted. By way of example, the vector wherein the polynucleotide or DNA construct of the invention is inserted may be a plasmid or a virus, which, once introduced in the host cell, may or may not integrate in the cell genome. This expression vector can be obtained by conventional methods.

Expression and cloning vectors usually contain a promoter that is recognized by the host organism and is operably linked to the coding sequence of interest. While the native promoter may be used, for most purposes heterologous promoters are preferred, as they generally permit greater transcription and higher yields.

Promoters suitable for use with prokaryotic hosts include the β-lactamase and lactose promoter systems, alkaline phosphatase, a tryptophan (trp) promoter system, hybrid promoters such as the tac promoter, the lacl promoter, the lacZ promoter, the T3 promoter, the T7 promoter, the arabinose promoter, the gpt promoter, the lambda PR promoter, the lambda PL promoter, promoters from operons encoding glycolytic enzymes such as 3-phosphoglycerate kinase (PGK), and the acid phosphatase promoter. In a preferred embodiment, said promoter is the T7 promoter. Their nucleotide sequences have been published, thereby enabling a skilled worker operably to ligate them to a sequence of interest using linkers or adaptors. Promoters for use in bacterial systems also will contain a Shine-Dalgarno (S.D.) sequence operably linked to the coding sequence. In certain cases, also, the host cell may be modified genetically to adjust concentrations of metabolite or inducer transporter proteins so that all cells in a culture will be induced equivalently.

According to the present invention, the polynucleotide sequence of the invention or genetic construct comprising the same is operably connected to a single promoter sequence.

Plasmid loss is the main cause of reduced recombinant protein productivity in plasmid-based systems. Several natural mechanisms exist to ensure plasmid survival in cell populations. The strategy most commonly used is to introduce a gene or genes that provide resistance to particular antibiotics into the plasmid. Selective pressure is then applied by supplementing the antibiotic to the culture medium. This approach can be ineffective if antibiotics are degraded or inactivated. Moreover, antibiotics are expensive, and their presence is undesirable in food and therapeutic products as well as in the exhausted culture broth that is discharged to wastewater treatment facilities of large scale fermentation operations. For these reasons, new strategies of plasmid maintenance that do not require the use of antibiotic resistance have been developed. They are based on the complementation of auxotrophic strains, operator-repressor titration, or toxin-antitoxin (TA) systems, among others.

According to another embodiment of the present invention, a toxin-antitoxin (TA) system is included in the genetic construct of the present invention. In a preferred embodiment, said system is a ccdBA TA system (Stieber, D. et al. 2008. Biotechniques 45(3): 432-34). The CcdB toxin (a growth repressor) is expressed from the bacterial chromosome under the control of a promoter strongly repressed by the CcdA antitoxin. CcdA is localized in the plasmid and its expression is under the control of a constitutive promoter. In *Escherichia coli* SE1 cells, when the antitoxin is present in the bacteria, the toxin is not produce and cells can grow normally. However, upon plasmid loss the toxin is induced causing cell death. This system is very appropriate for the steroselective production of (R)-3-quinuclidinol, because non-transformed *Escherichia coli* cells are able to reduce 3-quinuclidinone, generating 100% (S)-3-quinuclidinol. Thus, plasmid-free cells in a high-density culture generates (S)-3-quinuclidinol, which is an impurity for the purpose of this invention. Thus, the present invention also refers to plasmids or constructs containing GDH-Qred chimera and the CcdA antitoxin, which can be transcribed in the same direction or reversibly with respect to the GDH-Qred polypeptide (see Examples below).

In another aspect, the invention refers to a fusion or chimeric protein encoded by a polynucleotide sequence according to the invention. In a particular embodiment, said chimeric protein comprises the polypeptide sequence as shown in SEQ ID NO: 4. In another particular embodiment, said chimeric protein consists in the polypeptide sequence as shown in SEQ ID NO: 4.

Also, in another aspect, the invention relates to a host cell comprising the polynucleotide sequence according to the invention or a genetic construct or vector comprising the same or the fusion protein encoded thereof, wherein said polynucleotide sequence is expressible in said host cell. The cell is typically obtained by transformation, transfection or infection with a construct or vector provided by this invention. The transformed, transfected or infected cells can be obtained by conventional methods known by persons skilled in the art.

Host cells suitable for the expression of the polypeptide of the invention include, without being limited, mammal, plant, insect, fungal and microbial cells. In a preferred embodiment, said cell is a microbial cell. As used herein, the terms "microbial cell" and "microorganism" are herein indistinctively used and refer to any microscopic organism, including bacteria, fungi, microalgae, protists, and archaea. In a more preferred embodiment, said microbial cell is a bacterial cell. Bacterial cells include, without being limited to, Gram-positive bacterial cells such as species of the *Bacillus, Streptomyces* and *Staphylococcus* genus and Gram-negative bacterial cells such as cells of the *Escherichia* and *Pseudomonas* genus. In a more preferred embodiment said cell is *Escherichia coli.* Fungal cells preferably include cells of yeasts such as *Saccharomyces, Pichia pastoris* and *Hansenula polymorpha.* Insect cells include, without being limited to, *Drosophila* cells and Sf9 cells. Plant cells include, among others, cells of crop plants such as cereals, medicinal, ornamental or bulbous plants.

In another aspect, the invention relates to the use of a polynucleotide sequence according to the invention or genetic construct or vector according to the invention or the fusion protein encoded thereof or of a host cell according to the invention for the manufacture of (R)-3-quinuclidinol.

Thus, in another aspect, the invention refers to a method for the manufacture of (R)-3-quinuclidinol comprising
i) bringing into contact 3-quinuclidinone and glucose with a host cell according to the invention and
ii) recovering the (R)-3-quinuclidinol so produced.

In order to perform the method of the present invention, said host cell can be in the form of a culture broth, separated cells from the culture broth, i.e. whole cells, processed cells or immobilized forms thereof. In a preferred embodiment, said cells are whole cells.

As used herein, the term "processed cells" include treated cells such as, for example, dried cells, lyophilized cells or a cell lysate or extract thereof. Thus, the microbial cells according to the present invention can be employed as a wet pellet of cells of viable or dead organisms. Thus, said microbial cells can be employed as a washed pellet of cells which has been isolated by centrifugation and taken up in physiological saline. The term "cell lysate" or "cell extract" refers to a cell extract which includes the enzyme or cascade of enzymes that, when acting in a given sequence (e.g., in an enzymatic pathway) and proportion over a determined substrate, results in the preferential generation of a compound of interest. A compound of interest is typically a chemical entity (e.g., a small molecule), which can be used as an active pharmaceutical ingredient (API), chemical precursor, or intermediate, etc. Cells are lysed by any convenient method (physical or chemical) that substantially maintains enzyme activity, e.g. sonication, French press, and the like as known in the art. The lysate may be fractionated, particulate matter spun out, etc., or may be used in the absence of additional processing steps. The cell lysate may be further combined with substrates, co-factors and such salts, buffers, etc. as are required for enzyme activity.

The term "substrate," as used herein, is a compound or mixture of compounds capable of providing the required elements needed to synthesize a compound of interest.

The microorganisms identified by CECT numbers are listed in the catalogue from the "Colección Española de Cultivos Tipo" and are available from the CECT webpage (www.cect.org). The microorganisms identified by DSMZ numbers are listed in the catalogue of strains published by the "Deutsche Sammlung von Mikroorganismen und Zellkulturen" and are available from the DSMZ webpage (www.dsmz.de). The microorganisms identified by ATCC numbers are listed in the catalogue from the "American Type Culture Collection" and are available from the ATCC webpage (www.atcc.org).

The medium for growing microorganisms for use in the present invention is not restricted as far as they may grow and multiply therein. The medium normally includes, for example, carbon sources (saccharides; alcohols; organic acids; hydrocarbons; other carbon sources) and nitrogen sources (inorganic acid ammonium salts; organic acid ammonium salts; inorganic or organic nitrogen containing compounds such as yeast extract, meat extract, malt extract, soy extract, peptone, polypeptone, urea, etc.). In the medium, there may be incorporated appropriate amounts of those nutrients which are commonly employed in the cultivation of microorganisms, such as inorganic salts, trace metal salts and vitamins. Where necessary, factors that promote multiplication of a microorganism, factors which improve its ability to produce the object compound of the invention and compounds maintaining the pH of the medium (such as buffer substances) may be added.

The cultivation of the microorganism may be carried out under optimal conditions for the growth and multiplication of the microorganisms, for example at a pH of 5 - 9 and a temperature in the range of 20 - 50°C. The cultivation may be performed in aerobic or anaerobic conditions for one to five days.

In a first step of the method of the invention, the substrates 3-quinuclidinone and glucose are brought into contact with a host cell, in particular, with a microorganism or microbial cell according to the present invention.

There are well known techniques which can be employed according to the process of the invention in order to put into contact the substrates with the host cell.

As an example, said process comprises mixing or blending compound with a culture broth in order to conduct the reaction, i.e. whole cells are employed. Alternatively, the microbial cells are separated from the culture broth (for example by centrifugation, filtration, etc) resuspended, either as they are or after washing, in a buffer solution, water or the like, and then the substrates are added to the resultant cell suspension (resting cells) in order to conduct the reaction.

As mentioned above, whole cells are preferably used in the method of the present invention. Whole cell biocatalysts are generally more stable than purified enzymes. Biocatalytic reaction using whole cells is more stable than isolated enzyme reaction, and can reduce the cost of catalysts and cofactors. Indeed, as it is shown in the Examples accompanying the present invention, when using whole cells, there is no need to add a cofactor (e.g. NADPH) which is expensive and makes the process quite unsustainable. Additionally, since there is no need to lyse the cells, there will be no need of energy expenditure for breaking them. Also, the product will be easier to purify because the reaction solution will be less complex (cells can be simply separated from reaction mixture by centrifugation or filtration).

In another particular embodiment, whole cells or cell preparations as described above can be immobilized by known methods for use in the process of the present invention. As an example, the microorganisms can be physically inserted in a polymer matrix. A very wide variety of materials is used for the preparation of matrices of this type, using many different procedures, for example using polymers of unsaturated carboxylic acids with low molecular weight, such as acrylic acid and its derivatives.

According to a particular embodiment of the present invention, the biomass, i.e. the cells, can be reused in several bioconversion rounds. The reuse of the biocatalyst is of paramount importance because it represents a high cost saving to the industrial process. Indeed, smaller bioreactors for biomass production can be employed and there is no need to purchase industrial cell disruptors or homogenizers. Moreover, since both enzymes are expressed as a fusion protein in a single cell, only one type of cell will be needed to be grown in a bioreactor.

In many cases, a source of reducing power as NAD(P)H, glucose, sucrose, ethanol, isopropanol, methanol, salt of formate and the like are added to achieve better yields.

The reaction conditions are set in the margin where the activity of the microorganism or the preparation thereof is optimal. Thus, in a preferred embodiment, such conditions include a pH range of 5 - 9, a temperature of 20 - 50°C. In another preferred embodiment, the cell concentration is in the range of 1 - 200 g/I, on a wet cell basis

The optically active (R)-3-quinuclidinol produced according to the method of the invention can be recovered or harvested by conventional procedures such as, for example, separation of cells or cell preparations thereof (centrifugation, filtration) and purification (extraction with organic solvent, crystallization, recrystallization, chromatography, concentration and distillation). Any of the organic solvents (alcohols such as butanol; hydrocarbons such as hexane, cyclohexane, toluene; esters such as ethyl acetate; ketones; ethers and mixtures thereof) may be employed. Halogenated hydrocarbons such as chloroform and methylene chloride can be used.

The reactions may utilize a large scale reactor, small scale, or may be multiplexed to perform a plurality of simultaneous syntheses. Continuous reactions will use a feed mechanism to introduce a flow of reagents, and may isolate the end-product as part of the process. Batch systems are also of interest, where additional reagents may be introduced over time to prolong the period of time for active synthesis. A reactor may be run in any mode such as batch, extended batch, semi-batch, semi-continuous, fed-batch and continuous, and which will be selected in accordance with the application purpose.

The reactions may be of any volume, either in a small scale, usually at least about 1 ml and not more than about 15 ml, or in a scaled up reaction, where the reaction volume is at least about 15 ml, usually at least about 50 ml, more usually at least about 100 ml, and may be 500 ml, 1000 ml, or greater up to many thousands of litters of volume. Reactions may be conducted at any scale.

Various salts and buffers may be included, where ionic species are typically optimized with regard to product production. When changing the concentration of a particular component of the reaction medium, that of another component may be changed accordingly. Also, the concentration levels of components in the reactor may be varied over time. The adjuster of the thiol/disulfide oxidation/reduction potential may be dithiothreitol, ascorbic acid, glutathione and/or their oxidized forms. Other adjusters of the general redox potential may also be used.

In a semi-continuous operation mode, the reactor may be operated in dialysis, diafiltration batch or fed-batch mode. A feed solution may be supplied to the reactor through the same membrane or a separate injection unit. Synthesized product is accumulated in the reactor, and then is isolated and purified according to the usual method for purification after completion of the system operation. Alternatively, product can be removed during the process either in a continuous or discontinuous mode with the option of returning part of all of the remaining compounds to the reactor.

Where there is a flow of reagents, the direction of liquid flow can be perpendicular and/or tangential to a membrane. Tangential flow is effective for recycling ATP and for preventing membrane plugging and may be superimposed on perpendicular flow. Flow perpendicular to the membrane may be caused or effected by a positive pressure pump or a vacuum suction pump or by applying transmembrane pressure using other methods known in the art. The solution in contact with the outside surface of the membrane may be cyclically changed, and may be in a steady tangential flow with respect to the membrane. The reactor may be stirred internally or externally by proper agitation means.

The amount of product produced in a reaction can be measured in various fashions; for example, by enzymatic assays which produce a coloured or fluorometric product or by HPLC methods. One method relies on the availability of an assay which measures the activity of the particular product being produced.

The following examples are put forth so as to provide those of ordinary skill in the art with a complete disclosure and description of how to make and use the present invention, and are not intended to limit the scope of the invention or to represent that the experiments below are all or the only experiments performed. Efforts have been made to ensure accuracy with respect to numbers used (e.g., amounts, temperature, and the like), but some experimental errors and deviations may be present. Unless indicated otherwise, parts are parts by weight, molecular weight is weight average molecular weight, temperature is in degrees Centigrade, and pressure is at or near atmospheric.

### EXAMPLES

### Materials

Unless otherwise stated, chemicals were purchased from Sigma.

### Culture media

Luria-Bertrani (LB): 1.0 % tryptone (w/v), 0.5 % yeast extract, 0.5 % NaCl (pH 7.0). LB-agar plates: LB containg 1.5% agar. Auto-inducing media: 25mM Na₂HPO₄, 25mM KH₂PO₄, 50mM NH₄Cl, 5mM Na₂SO₄, 2mM MgSO₄, 0.5% glycerol, 0.05% glucose, 0.2% lactose, pH 7.0

### Molecular biology products

Restriction enzymes, dNTPs mix, read-proof Taq polymerases and T4 DNA ligase were purchased from Promega Corp. Synthetic genes were designed and purchased from GeneArt ® (LifeTechnologies™).

### Bacterial strains and culture conditions

Cells were grown in glass Erlenmeyer flasks at 25-37°C under vigorous agitation (200-250 rpm) in thermostated shakers.

### Bacterial strains

The bacterial strains used in this invention are listed in Table 1.

**Table 1. Bacterial strains**

| **Strain** | **Provider** | **Use** |
|---|---|---|
| *Bacillus subtilis* QM 5115 | LGC Standards | Glucose 1-dehydrogenase cloning |
| *Escherichia coli* DH5α | Laboratory collection | Recombinant plasmids selection |
| *Escherichia coli* | EMDMillipore | Protein expression |
| BL21(D3) | | |
| *Escherichia coli* | EMDMillipore | Protein expression |
| Rosetta (D3)pLys | | |
| *Escherichia coli* | DelphiGenetics | Recombinant plasmids selection |
| CYS21 | | |
| *Escherichia coli* | DelphiGenetics | Protein expression |
| SE1 | | |

### Plasmid vectors

The vectors used in this patent are listed in Table 2.

**Table 2. Cloning vectors.**

| **Name** | **provider** | **Antibiotic resistance** | **Origin of replication** | **Plasmid compatibility** | **purpose** |
|---|---|---|---|---|---|
| pET21d(+) | EMD Millipore (Novagen) | Ampicillin | pBR322 | RSF | cloning |
| pETDuet-1 | EMD Millipore (Novagen) | Ampicillin | pBR322 | pBR322 | cloning |
| pRSF1b | EMD Millipore (Novagen) | Kanamycin | RSF | pBR322 | cloning |
| pGetStabyA | DelphiGenetics | Ampicillin | | - | *ccd*A template |

### Oligonucleotides

Oligonucleotides were designed and purchased from Sigma.

The oligonucleotides used for cloning purposes are listed in Table 3.

**Table 3. Oligonucleotides used for cloning purposes.**

| Name | Sequence (5'-3') (restriction sites underlined) | Amplified gene | SEQ ID NO |
|---|---|---|---|
| GDH-FW | CCATGGCCTATACAGATTTAAAAG | *Bacillus megaterium* GDH | SEQ ID NO: 5 |
| GDH-RV | GGATCCTTAGCCTCTTCCTGCTTGG | *Bacillus megaterium* GDH | SEQ ID NO: 6 |
| Get-FW | GGATCCCGTAGGCGGTCACGACTTTG | *Escherichia coli ccd*A | SEQ ID NO: 7 |
| Get-RV | GGATCCTAGTAACGGCCGCCAGTGTG | *Escherichia coli ccd*A | SEQ ID NO: 8 |

### Cloning strategy

### Bacillus megaterium glucose 1-dehydrogenase in pRSF1b (GDH-pRSF)

Glucose 1-dehydrogenase gene (YP_003563827) was amplified with oligos GDH-FW (forward) and GDH-RV (reverse) (see Table 3) using purified genomic DNA from *Bacillus megaterium* QM B1551 (ATCC 12872) as template. The amplified fragment was digested with Ncol and *Bam*HI and cloned in the corresponding restriction sites of pRSF1b. Ligation reactions were transformed in *Escherichia coli* DH5α chemically competent cells and transformants selected on LB-kanamycin (0.05mg/ml) plates. Transformed clones were tested by colony PCR. Plasmid DNA was purified from positive clones and sequence integrity of the 786-bp coding region checked by automated fluorescence sequencing.

### Rhodotorula rubra NADPH-dependent 3-quinuclidinone reductase (Qred-pET21d and Qred-pDuet)

NADPH-dependent 3-quinuclidinone reductase gene from *Rhodotorula mucilaginosa* (GenBank: BAH28833.1) was designed and synthesized by GeneArt® (Lifetechnologies™) and its codon usage optimized for expression in *Escherichia coli.* The coding region was cloned in pMA-T vector and subsequently subcloned in pET21d(+) and pETDuet-1 between *Bam*HI and Ncol restriction sites. Ligation reactions were transformed in *Escherichia coli* DH5α chemically competent cells and transformants selected on LB-ampicillin (0.05mg/ml) plates. Transformed clones were tested by colony PCR. Plasmid DNA was purified from positive clones and sequence integrity of the 819-bp coding region checked by automated fluorescence sequencing.

### Bacillus megaterium glucose 1-dehydrogenase and Rhodotorula rubra NADPH-dependent 3-quinuclidinone reductase chimeric construction (GDH-linker-Qred-pRSF)

A chimeric construction containing in-frame fused *Bacillus megaterium* glucose 1-dehydrogenase (GDH) and *Rhodotorula rubra* NADPH-dependent 3-quinuclidinone reductase (Qred) linked by a 15-mer amino acid linker was designed and synthesized by GeneArt® (Lifetechnologies) and its codon usage optimized for expression in *Escherichia coli.* The coding region was cloned in pMA-RQ vector and subsequently subcloned in pRSF1b between *Bam*HI and Ncol restriction sites (GDH-linker-Qred-pRSF). Ligation reactions were transformed in *Escherichia coli* DH5α cells and transformants selected on LB-agar kanamycin plates. Transformed clones were tested by colony PCR. Plasmid DNA was purified from positive clones and sequence integrity of the 1644-bp coding region checked by automated fluorescence sequencing (SEQ ID NO: 11). When expressed, this coding region gave rise to a fusion or chimeric protein comprising the polypeptide sequence as shown in SEQ ID NO: 4.

### Bacillus megaterium glucose 1-dehydrogenase and Rhodotorula rubra NADPH-dependent 3-quinuclidinone reductase chimeric construction containing Escherichia coli ccdA antitoxin (GDH-linker-Qred-pRSF-ccdA)

A ccdBA TA system has been used in the present invention. The CcdB toxin (a growth repressor) is expressed from the bacterial chromosome under the control of a promoter strongly repressed by the CcdA antitoxin. CcdA is localized in the plasmid and its expression is under the control of a constitutive promoter. In *Escherichia coli* SE1 cells, when the antitoxin is present in the bacteria, the toxin is not produce and cells can grow normally. However, upon plasmid loss the toxin is induced causing cell death. This system is very appropriate for the steroselective production of (R)-3-quinuclidinol, because non-transformed *Escherichia coli* cells are able to reduce 3-quinuclidinone, generating 100% (S)-3-quinuclidinol. Thus, plasmid-free cells in a high-density culture generates (S)-3-quinuclidinol, which is an impurity for the purpose of this invention.

Plasmids coding for GDH-linker-Qred chimera and the CcdA antitoxin, which is transcribed in the same direction or reversibly with respect to the GDH-linker-Qred polypeptide (plasmids GDH-linker-Qred→ccdA or GDH-linker-Qred←ccdA respectively) have been constructed. To make these constructions, *ccd*A was amplified with oligos Get-FW (forward) and Get-RV (reverse) using pGetStaby plasmid as template. The purified fragment was digested with *Bam*HI and non-oriented cloned in GDH-linker-Qred-pRSF digested with *Bam*HI. Recombinant clones of both orientations were analyzed to check the potential influence of *ccd*A transcription orientation in the plasmid maintenance (SEQ ID NO: 9 and SEQ ID NO: 10). When expressed, these coding regions gave rise to a fusion or chimeric protein comprising the polypeptide sequence as shown in SEQ ID NO: 4.

### Protein expression experiments

*Bacillus megaterium* glucose 1-dehydrogenase in pRSF1b (GDH-pRSF) construction was transformed in *Escherichia coli* BL21(D3) and Rosetta (D3) cells for protein expression and transformants were selected on LB-agar kanamycin plates.

*Rhodotorula rubra* 3-quinuclidinone reductase in pET21d(+) (Qred-pET21d) or in pDuet1 (Qred-pDuet) constructions were transformed in chemically competent *Escherichia coli* BL21(D3) and Rosetta (D3) cells for protein expression and transformants were selected on LB-agar ampicillin plates.

*Bacillus megaterium* glucose 1-dehydrogenase in pRSF1b (GDH-pRSF) and *Rhodotorula rubra* 3-quinuclidinone reductase in pET21d(+) (Qred-pET21d) or in pDuet1 (Qred-pDuet) were co-transformed in chemically competent *Escherichia coli* BL21(D3) and Rosetta (D3) cells for protein co-expression and transformants were selected on LB-agar kanamycin-ampicilin plates (0.05 mg/ml each).

The chimeric construction containing in-frame fused *Bacillus megaterium* glucose 1-dehydrogenase (GDH) and *Rhodotorula rubra* NADPH-dependent 3-quinuclidinone reductase (Qred) linked by a 15-mer amino acid linker (GDH-linker-Qred) was transformed in chemically competent *Escherichia coli* BL21(D3) and Rosetta (D3) cells for protein expression and transformants were selected on LB-agar kanamycin plates.

The chimeric constructions containing in-frame fused *Bacillus megaterium* glucose 1-dehydrogenase (GDH) and *Rhodotorula rubra* NADPH-dependent 3-quinuclidinone reductase (Qred) linked by a 15-mer amino acid linker (GDH-linker-Qred) and *ccd*A antitoxin were transformed in chemically competent *Escherichia coli* SE1 cells for protein expression and transformants were selected on LB-agar plates.

*Escherichia coli* BL21(D3) and Rosetta (D3) cells transformed with Qred-pET21d or Qred-pDuet1 were pre-inoculated and grown overnight at 26, 30 or 37°C in 2-ml LB-ampicillin tubes. *Escherichia coli* BL21(D3) and Rosetta (D3) cells transformed with GDH-pRSF or GDH-linker-Qred-pRSF were pre-inoculated and grown overnight at 26, 30 or 37°C in 2-ml LB-kanamycin tubes. *Escherichia coli* BL21(D3) and Rosetta (D3) cells co-transformed with GDH-pRSF and Qred-pET21d or Qred-pDuet1 were pre-inoculated and grown overnight at 26, 30 or 37°C in 2-ml LB-kanamycin-ampicillin tubes. Cells were diluted from 100 to 1000-fold in 250-ml Erlenmeyer flasks containing 50-ml of fresh LB medium with the appropriate antibiotics and grown at 26, 30 or 37°C till they reached optical density at 600 nm (OD₆₀₀ₙₘ) of 0.3-1.5. Protein expression was induced by the addition of 0.2-2mM isopropyl-b-D-1-thiogalactopyranoside (IPTG) at 26, 30 or 37°C. 1-ml aliquots of the cultures were withdrawn at 0, 2, 3 and 18h and stored at -80°C for further analysis of recombinant protein expression.

*Escherichia coli* SE1 cells transformed with GDH-linker-Qred-pRSF-*ccd*A were pre-inoculated and grown overnight at 26, 30 or 37°C in 2-ml LB-kanamycin tubes. Cells were diluted from 100 to 1000-fold in 250-ml Erlenmeyer flasks containing 50-ml of fresh LB medium and grown at 26, 30 or 37°C till they reached optical density at 600 nm (OD₆₀₀ₙₘ) of 0.3-1.5. Protein expression was induced by the addition of 0.2-2mM isopropyl-b-D-1-thiogalactopyranoside (IPTG) at 26, 30 or 37°C. 1-ml aliquots of the cultures were withdrawn at 0, 2, 3 and 18h and stored at -80°C for further analysis of recombinant protein expression.

For protein auto-induction, pre-inocula were grown overnight as described above. Cells were diluted from 100 to 1000-fold in 250-ml Erlenmeyer flasks containing 50-ml of fresh auto-inducing media containing the appropriate antibiotics and grown at 26, 30 or 37°C for 18 hours. 1-ml aliquots of the cultures were withdrawn at 0, 2, 3, 4, 5, 6 and 18h after reinoculation and stored at -80°C for further analysis of recombinant protein expression. After 18-hour growth cells were harvested, processed (as described below) and used as biocatalysts.

### Protein expression:

Cells transformed with one or two plasmids were grown and protein induced as described in the "Protein expression experiments section". Protein expression was checked by SDS-polyacrylamide gel electrophoresis (SDS-PAGE). Identity of induced recombinant proteins was confirmed by matrix-assisted laser desorption/ionization-time of flight (MALDI-TOF) of gel-excised protein bands digested with trypsin.

### Biocatalyst preparation

IPTG induced or auto-induced cells were centrifuged and cell pellet was weighted (wet cell weight, wcw) and ressuspended in 4-10 ml of phosphate buffer (25-100mM phosphate buffer, pH 5-8) per gram of wcw. Ressupended cells ("whole cell assays"), or pre-treated with permeabilized agents cells ("permeabilized whole cell assays") or lysed cells ("crude extract assays") were used as biocatalyst.

In "permeabilized whole cell assays", ressuspended cells were pre-treated with 0.1-1% toluene for 1 hour at 30°C and then used as biocatalyst in the 3-quinuclidinol synthesis reaction.

In "crude extract assays", ressuspended cells were disrupted by sonication. Non-centrifuged crude extracts were used as biocatalyst in the 3-quinuclidinol synthesis reaction.

### 3-quinuclidinol synthesis reaction

5-mL reaction mixtures in 15 mL tubes contained 4 ml of 2.5-20 mg/ml of 3-quinuclidinone, 1-10 mg/ml of glucose and 0-16 mg/ml of NADPH in 25-100mM phosphate buffer, pH 5-8 and 1ml of biocatalyst ("whole cells", "permeabilized whole cells" or "crude extracts"). Reaction mixtures were agitated at 20-37°C for 4-96 hours. Reaction scale-up was performed keeping the components' proportions in Erlenmeyer's flasks with magnetic stirring.

### Sample analyses

1-ml aliquots of the reaction mixture were withdrawn at different times, centrifuged at 12000xg for 5 minutes. The supernatant was differentially treated for HPLC/MS analysis or chiral gas chromatography (GC).

For the screening of experimental conditions, a HPLC/MS method was set-up in order to determine conversion rates. Supernatant from samples were diluted 1000-fold in mQ H₂O and analyzed by HPLC using a HILIC column [XBridge™ HILIC (3.5 µm, 2.1 x 100 mm); mobile phase (acetonitrile/ammonium acetate 10 mM pH 4 = 80/20); column temperature: 30°C; flow rate: 0.3 ml/min; detection: single quadrupole equipped with an ESI interface operating in positive mode, SIM (m/z) 3-quinuclidinone (126), 3-quinuclidinol (128)]. Approximate retention time: 3-quinuclidinone 2.3 min; (R,S)-3-quinuclidinol 3 min.

Yields and enantiomeric purity determination for the bioconversion of 3-quinuclidinone into (R)-3-quinuclidinol were carried out by chiral GC. 1-ml supernatant from samples were evaporated at 80°C under a nitrogen stream for 30 minutes in 1.5 ml HPLC-type glass vials. The dried pellet was ressuspended in 150µl of methanol, butanol or isobutanol. The samples were centrifuged at 12000xg for 1 minute to eliminate the non-soluble fraction and analyzed by CG using a chiral column [Cyclosil B (30 m x 0.25 mm x 0.25 µm); flow 1.4 ml/min; oven program: 140°C for 18 min, 20°C/min to 240°C for 4 min), injector 280°C, detection: FID 250°C). Approximate retention time: 3-quinuclidinone 8.8 min; (S)-3-quinuclidinol 14.2 min; (R)-3-quinuclidinol 14.8 min.

### Work-up for product isolation

Samples were centrifuged for 30 minutes at 3000-6000 rpm at room temperature. Supernatant was treated with HCl 5N till pH<2.0. The acidified sample was vacuum filtered on PVDF filter. The flow-through was treated with NaOH till pH>12. This was rotavaporated to dryness with the help of acetonitrile. Dry pellet was ressuspended in dichloromethane and vacuum filtered. Flow-through was recovered and rotavaporated to dryness. Toluene was added and the mixture was warmed till complete solubility. 3-quinuclidinol crystals appeared after slow cooling of the solution. Crystals were vacuum dried and weighted to calculate purification yield. Powdered product was analyzed by infrared spectrophotometry, polarimetry, nuclear magnetic resonance. Characterization of isolated product was assessed by infrared spectroscopy (IR), chiral GC, nuclear magnetic resonance (NMR), polarimetry and mass spectroscopy (MS) scan. Characterization data matched with the standard.

### Results

*Escherichia coli* BL21(D3) or Rosetta(D3)pLys cells transformed with Qred-pET21d or Qred-pDuet and GDH-pRSF were grown and proteins induced by the addition of 0.2-2mM IPTG or in auto-inducing media. 18-hours induced cultures were centrifuged and a fraction of cells were ressuspended in phosphate buffer for "whole cells assays". Another aliquot was sonicated to obtain the crude extract for the "crude extract assays". Biocatalysts were incubated with 3-quinuclidinone and glucose in phosphate buffer at 30°C for 48-96 hours. Typical results are shown in Table 4.

**Table 4. % of conversion and enantiopurity of biocatalytical conversion of 3-quinuclidinone to (R)-3-quinuclidinol**

| **Cells** | **Plasmids** | **Assay** | **% conversion** | **% (R)-3-quinuclidinol** |
|---|---|---|---|---|
| *E.coli* BL21(D3) | Qred-pET21d; GDH-pRSF | Whole cells | <50 | <85 |
| *E.coli* BL21(D3) | Qred-pDuet; GDH-pRSF | Whole cells | <20 | <65 |
| *E.coli* BL21(D3) | Qred-pET21d; GDH-pRSF | Crude extracts | <50 | <85 |
| *E.coli* BL21 (D3) | Qred-pDuet; GDH-pRSF | Crude extracts | <20 | <65 |
| *E.coli* Rosetta (D3) pLys | Qred-pET21d; GDH-pRSF | Whole cells | <30 | <85 |
| *E.coli* Rosetta (D3) pLys | Qred-pDuet; GDH-pRSF | Whole cells | <20 | <85 |
| *E.coli* Rosetta (D3) pLys | Qred-pET21d; GDH-pRSF | Crude extracts | <30 | <85 |
| *E.coli* Rosetta (D3) pLys | Qred-pDuet; GDH-pRSF | Crude extracts | <20 | <85 |

*Escherichia coli* BL21(D3) or Rosetta(D3)pLys transformed with GDH-linker-Qred-pRSF and *Escherichia coli* SE1 cells transformed with GDH-linker-Qred-ccdA-pRSF were grown and chimeric protein induced by the addition of 0.2-2mM IPTG or in auto-inducing media. 18-hours induced cultures were centrifuged and a fraction of cells were ressuspended in phosphate buffer for "whole cells assays". Another aliquot was sonicated to obtain the crude extract for the "crude extract assays". Biocatalysts were incubated with 3-quinuclidinone and glucose in phosphate buffer at 30°C for 4-96 hours. Typical results are shown in Table 5.

**Table 5. % of conversion and enantiopurity of biocatalytical conversion of 3-quinuclidinone to (R)-3-quinuclidinol**

| **Cells** | **Plasmids** | **Assay** | **% conversion** | **% (R)-3-quinuclidinol** |
|---|---|---|---|---|
| *E.coli* BL21(D3) | GDH-linker-Qred-pRSF | Whole cells | >95 | >97 |
| *E.coli* BL21 (D3) | GDH-linker-Qred-pRSF | Crude extracts | >98 | >98 |
| *E.coli* Rosetta (D3) pLys | GDH-linker-Qred-pRSF | Whole cells | >95 | >97 |
| *E.coli* Rosetta (D3) pLys | GDH-linker-Qred-pRSF | Crude extracts | >98 | >98 |
| *E.coli* SE1 | GDH-linker-Qred-pRSF-ccdA | Whole cells | >99 | >99 |
| *E.coli* SE1 | GDH-linker-Qred-pRSF-ccdA | Crude extracts | >99 | >99 |

### Biomass reuse

Whole cells were incubated with 3-quinuclidinone and glucose in 25-100mM phosphate buffer, pH 5-8, per gram of wet cell weight (wcw) at 20-50°C and aliquots were withdrawn at different time points for HPLC/MS analysis. After 4-hour incubation, reaction solution was centrifuged and cells were incubated again with the same reaction buffer. Aliquots were withdrawn and bioconversion checked by HPLC/MS. Biomass could be reused twice (in total three bioconversion rounds).

### Discussion

Whole cells and crude extracts of *Escherichia coli* cells expressing GDH-linker-Qred chimera have shown to be capable of quantitatively transforming 3-quinuclidinone into (R)-3-quinuclidinol (conversion rate >99%; ee>99%).

## Claims

1. An isolated polynucleotide sequence encoding a chimeric fusion protein comprising a glucose 1-dehydrogenase amino acid sequence or a functionally equivalent variant thereof and a NADPH-dependent 3-quinuclidinone reductase amino acid sequence or a functionally equivalent variant thereof.

2. Polynucleotide sequence according to claim 1, wherein said glucose 1-dehydrogenase sequence is a *Bacillus megaterium* glucose 1-dehydrogenase sequence.

3. Polynucleotide sequence according to claim 1, wherein said NADPH-dependent 3-quinuclidinone reductase sequence is a *Rhodotorula rubra* NADPH-dependent 3-quinuclidinone reductase sequence.

4. Polynucleotide sequence according to any one of claims 1 to 3, wherein the NADPH-dependent 3-quinuclidinone reductase sequence is linked to the glucose 1-dehydrogenase sequence by a linker sequence.

5. Polynucleotide sequence according to claim 4, wherein said linker sequence is an amino acid sequence of 5 to 25 amino acids length.

6. Polynucleotide sequence according to claim 5, wherein said linker sequence is of 15 amino acids length.

7. Polynucleotide sequence according to any one of claims 5 or 6, wherein said linker sequence is a sequence comprising two or more Thr and Pro pairs.

8. A genetic construct comprising a polynucleotide according to any one of claims 1 to 7.

9. A fusion protein encoded by a polynucleotide sequence according to any one of claims 1 to 7 or a genetic construct according to claim 8.

10. A host cell comprising a polynucleotide sequence according to any one of claims 1 to 7 or a genetic construct of claim 8 or a fusion protein according to claim 9, wherein said polynucleotide sequence is expressible in said host cell.

11. Host cell according to claim 10, wherein said cell is a microbial cell.

12. Use of a polynucleotide according to any one of claims 1 to 7, or a genetic construct according to claim 8, or a fusion protein according to claim 9 or a host cell according to claim 10 or 11 for the manufacture of (R)-3-quinuclidinol.

13. A method for the manufacture of (R)-3-quinuclidinol comprising
i) bringing into contact 3-quinuclidinone and glucose with a host cell according to claims 10 or 11; and
ii) recovering the (R)-3-quinuclidinol so produced.

14. Method according to claim 13, wherein said host cell is in the form of a culture broth, separated cells from the culture broth, treated preparations of cells or immobilized forms thereof.

15. Method according to any one of claims 13 or 14, wherein when said cells are treated preparation of cells NADPH is added in step i).


SEQUENCE LISTING

<110>  INTERQUIM, S.A.

<120>  STEREOSELECTIVE PRODUCTION OF (R)-3-QUINUCLIDINOL

<130>  P2679EP00

<160>  13

<170>  PatentIn version 3.5

<210>  1
<211>  786
<212>  DNA
<213>  Bacillus megaterium

<400>  1
atggcctata cagatttaaa agataaagta gtagttgtaa caggcggatc aaaaggattg      60

ggtcgcgcaa tggccgttcg ttttggtcaa gagcagtcaa aagtggttgt aaactaccgc     120

agcaatgaag aagaagcgct agaagtaaaa aaagaaattg aacaagctgg cggccaagca     180

attattgttc gaggcgacgt aacaaaagag gaagacgttg tgaatcttgt agagacagct     240

gttaaagagt ttggcacatt agacgttatg attaacaatg ctggtgttga aaacccggtt     300

ccttcacatg aattatcgtt agaaaactgg aatcaagtaa tcgatacaaa cttaacaggc     360

gcgtttttag gaagccgcga agcgattaaa tattttgttg aaaatgatat taaaggaaac     420

gttattaaca tgtccagcgt tcacgagatg attccttggc cactatttgt tcactatgca     480

gcaagtaaag gcggtatgaa actaatgaca gaaacattgg ctcttgaata tgcgccaaaa     540

ggtatccgcg taaataacat tggaccaggc gcgatcgata cgccaatcaa cgctgaaaaa     600

ttcgcagatc cggaacagcg tgcagacgta gaaagcatga ttccaatggg ctacatcggc     660

aacccggaag aaattgcatc agttgcagca ttcttagcat cgtcacaagc aagctacgta     720

acaggtatta cactatttgc tgatggcggt atgacaaaat atccttcttt ccaagcagga     780

agaggc                                                                786


<210>  2
<211>  819
<212>  DNA
<213>  Rhodotorula rubra

<400>  2
atgggcagca gcccgagtga tggtccgttt ccgaaagcaa caccgcagct gccgaatagc      60

gtttttgata tgtttagcat gaaaggcaaa gtgaccgcaa ttaccggtgg tggtggcggt     120

attggttttg cagcagcaga agcaattgcc gaagccggtg gtgatgttgc actgctgtat     180

cgtagcgcac cgaatatgga agaacgtagc gcagaactgg ccaaacgttt tggtgttaaa     240

gttaaagcct atcagtgcga agtgaccgaa catgaaagcg ttaaacaggc aattgaagcc     300

gtggaaaaag attttggtcg tctggattgt tatattgcaa atgcaggcgg tggtgttccg     360

ggtagcatta acccggatta tccgctggaa gcatggcata aaacccagag cgttaatctg 420

catagcacct tttatgcagc acgtgaatgt gcacgtatct ttaaagcaca gggtagcggt 480

agctttattg caaccaccag tattagcgca cgtattgtta atgttccgta tgatcagcct 540

gcatacaata gcagcaaagc agcagttgtt catttttgtc gtagcctggc acgtgattgg 600

cgtaattttg cacgtgttaa taccattagc cctggctttt ttgatacccc gatgggtccg 660

agcgataaag cagttgaaga tgttctgtat cagaaaagcg ttctgggtcg tgccggtgat 720

gttaaagaac tgaaagcagc atatctgtat ctggcaagca atgcaagcac ctataccacc 780

ggtgccgatc tgctgattga tggtggttat tgtctgacc 819

<210> 3
<211> 15
<212> PRT
<213> Artificial Sequence

<220>
<223> linker sequence

<400> 3

Pro Thr Pro Thr Pro Thr Pro Thr Pro Thr Pro Thr Pro Thr Pro
1               5                   10                  15

<210> 4
<211> 548
<212> PRT
<213> Artificial Sequence

<220>
<223> Chimeric protein GDH-linker-Qred

<400> 4

Met Ala Tyr Thr Asp Leu Lys Asp Lys Val Val Val Val Thr Gly Gly
1               5                   10                  15

Ser Lys Gly Leu Gly Arg Ala Met Ala Val Arg Phe Gly Gln Glu Gln
            20                  25                  30

Ser Lys Val Val Val Asn Tyr Arg Ser Asn Glu Glu Glu Ala Leu Glu
        35                  40                  45

Val Lys Lys Glu Ile Glu Gln Ala Gly Gly Gln Ala Ile Ile Val Arg
    50                  55                  60

Gly Asp Val Thr Lys Glu Glu Asp Val Val Asn Leu Val Glu Thr Ala
65                  70                  75                  80

Val Lys Glu Phe Gly Thr Leu Asp Val Met Ile Asn Asn Ala Gly Val
                85                  90                  95

Glu Asn Pro Val Pro Ser His Glu Leu Ser Leu Glu Asn Trp Asn Gln
            100                 105                 110

Val Ile Asp Thr Asn Leu Thr Gly Ala Phe Leu Gly Ser Arg Glu Ala
        115                 120                 125

Ile Lys Tyr Phe Val Glu Asn Asp Ile Lys Gly Asn Val Ile Asn Met
    130                 135                 140

Ser Ser Val His Glu Met Ile Pro Trp Pro Leu Phe Val His Tyr Ala
145                 150                 155                 160

Ala Ser Lys Gly Gly Met Lys Leu Met Thr Glu Thr Leu Ala Leu Glu
                165                 170                 175

Tyr Ala Pro Lys Gly Ile Arg Val Asn Asn Ile Gly Pro Gly Ala Ile
            180                 185                 190

Asp Thr Pro Ile Asn Ala Glu Lys Phe Ala Asp Pro Glu Gln Arg Ala
        195                 200                 205

Asp Val Glu Ser Met Ile Pro Met Gly Tyr Ile Gly Asn Pro Glu Glu
    210                 215                 220

Ile Ala Ser Val Ala Ala Phe Leu Ala Ser Ser Gln Ala Ser Tyr Val
225                 230                 235                 240

Thr Gly Ile Thr Leu Phe Ala Asp Gly Gly Met Thr Lys Tyr Pro Ser
                245                 250                 255

Phe Gln Ala Gly Arg Gly Pro Thr Pro Thr Pro Thr Pro Thr Pro Thr
            260                 265                 270

Pro Thr Pro Thr Pro Ser Ser Pro Ser Asp Gly Pro Phe Pro Lys Ala
        275                 280                 285

Thr Pro Gln Leu Pro Asn Ser Val Phe Asp Met Phe Ser Met Lys Gly
    290                 295                 300

Lys Val Thr Ala Ile Thr Gly Gly Gly Gly Gly Ile Gly Phe Ala Ala
305                 310                 315                 320

Ala Glu Ala Ile Ala Glu Ala Gly Gly Asp Val Ala Leu Leu Tyr Arg
                325                 330                 335

Ser Ala Pro Asn Met Glu Glu Arg Ser Ala Glu Leu Ala Lys Arg Phe
            340                 345                 350

Gly Val Lys Val Lys Ser Tyr Gln Cys Glu Val Thr Glu His Glu Ser

355                 360                 365

Val Lys Gln Ala Ile Glu Ala Val Glu Lys Asp Phe Gly Arg Leu Asp
    370                 375                 380

Cys Tyr Ile Ala Asn Ala Gly Gly Gly Val Pro Gly Ser Ile Asn Pro
385                 390                 395                 400

Asp Tyr Pro Leu Glu Ala Trp His Lys Thr Gln Ser Val Asn Leu His
                405                 410                 415

Ser Thr Phe Tyr Ala Ala Arg Glu Cys Ala Arg Ile Phe Lys Ala Gln
            420                 425                 430

Gly Ser Gly Ser Phe Ile Ala Thr Thr Ser Ile Ser Ala Arg Ile Val
        435                 440                 445

Asn Val Pro Tyr Asp Gln Pro Ala Tyr Asn Ser Ser Lys Ala Ala Val
    450                 455                 460

Val His Phe Cys Arg Ser Leu Ala Arg Asp Trp Arg Asn Phe Ala Arg
465                 470                 475                 480

Val Asn Thr Ile Ser Pro Gly Phe Phe Asp Thr Pro Met Gly Pro Ser
                485                 490                 495

Asp Lys Ala Val Glu Asp Val Leu Tyr Gln Lys Ser Val Leu Gly Arg
            500                 505                 510

Ala Gly Asp Val Lys Glu Leu Lys Ala Ala Tyr Leu Tyr Leu Ala Ser
        515                 520                 525

Asn Ala Ser Thr Tyr Thr Thr Gly Ala Asp Leu Leu Ile Asp Gly Gly
    530                 535                 540

Tyr Cys Leu Thr
545

<210>  5
<211>  24
<212>  DNA
<213>  Artificial Sequence

<220>
<223>  Primer FW for cloning Bacillus megaterium GDH gene

<400>  5
ccatggccta tacagattta aaag                                              24

<210>  6
<211>  25

<212>  DNA
<213>  Artificial Sequence

<220>
<223>  Primer RV for cloning Bacillus megaterium GDH gene

<400>  6
ggatccttag cctcttcctg cttgg                                            25


<210>  7
<211>  26
<212>  DNA
<213>  Artificial Sequence

<220>
<223>  Primer FW for cloning Escherichia coli ccdA

<400>  7
ggatcccgta ggcggtcacg actttg                                           26


<210>  8
<211>  26
<212>  DNA
<213>  Artificial Sequence

<220>
<223>  Primer RV for cloning Escherichia coli ccdA

<400>  8
ggatcctagt aacggccgcc agtgtg                                           26


<210>  9
<211>  2087
<212>  DNA
<213>  Artificial Sequence

<220>
<223>  DNA sequence of GDH-linker-Qred chimeric-ccdA construction

<400>  9
atggcatata ccgacctgaa agataaagtt gttgttgtta ccggtggtag caaaggtctg      60

ggtcgtgcaa tggcagttcg ttttggtcaa gaacagagca aagttgtggt taattatcgc     120

agcaatgaag aagaagccct ggaagtcaaa aaagaaattg aacaggcagg cggtcaggca     180

attattgttc gtggtgatgt taccaaagaa gaggacgttg ttaatctggt tgaaaccgca     240

gttaaagaat ttggcaccct ggatgtgatg attaacaatg ccggtgttga aaatccggtt     300

ccgagccatg aactgagcct ggaaaattgg aatcaggtga ttgataccaa tctgaccggt     360

gcatttctgg gtagccgtga agccattaaa tactttgtgg aaaacgacat caaaggcaac     420

gtgattaata tgagcagcgt gcatgaaatg attccgtggc ctctgtttgt tcattatgca     480

gcaagcaaag gtggtatgaa actgatgacc gaaaccctgg cactggaata tgcaccgaaa     540

ggtattcgtg ttaataacat tggtccgggt gcaattgata ccccgattaa tgcagaaaaa     600

tttgcagatc cggaacagcg tgcagatgtt gaaagcatga ttccgatggg ttatattggt     660

aacccggaag aaattgcaag cgttgcagcc tttctggcaa gcagccaggc aagctatgtg 720

accggtatta ccctgtttgc agatggtggc atgaccaaat atccgagctt tcaggcaggt 780

cgtggtccga ccccgacacc gactcctacc cctacaccaa ccccgacccc gtcaagcccg 840

agtgatggtc cgtttccgaa agcaacaccg cagctgccga atagcgtttt tgatatgttt 900

agcatgaaag gcaaagtgac cgcaattaca ggtggtggtg gcggtattgg ttttgcagca 960

gcagaagcaa ttgccgaagc aggcggtgat gttgcactgc tgtatcgtag cgcaccgaat 1020

atggaagaac gtagcgcaga actggccaaa cgttttggtg ttaaagttaa aagctatcag 1080

tgcgaagtga ccgaacatga aagcgttaaa caggcaattg aagccgtgga aaaagatttt 1140

ggtcgtctgg attgttatat tgcaaatgcg ggtggcggtg ttccgggtag cattaacccg 1200

gattatccgc tggaagcatg gcataaaacc cagagcgtta atctgcatag caccttttat 1260

gcagcccgtg aatgtgcacg tatctttaaa gcacagggta gcggtagctt tattgcaacc 1320

accagtatta gcgcacgtat tgttaatgtt ccgtatgatc agcctgcata caatagcagc 1380

aaagcagcag ttgttcattt ttgtcgtagc ctggcacgtg attggcgtaa ttttgcacgt 1440

gtgaatacca tttcaccggg tttttttgat acaccgatgg gtccgagcga taaagcagtt 1500

gaagatgttc tgtatcagaa aagcgttctg ggacgtgccg gtgatgtgaa agaactgaaa 1560

gcagcatatc tgtatctggc cagcaatgca agcacctata ccacaggtgc cgatctgctg 1620

attgatggtg gttattgtct gacctaagga tcccgtaggc ggtcacgact ttgcgaagca 1680

aagtctagtg agtatactca agcattgagt ggcccgccgg aggcaccgcc ttgcgctgcc 1740

cccgtcgagc cggttggtca ccaaaaggga ggggcaggca tgaagcagcg tattacagtg 1800

acagttgaca gcgacagcta tcagttgctc aaggcatatg atgtcaatat ctccggtctg 1860

gtaagcacaa ccatgcagaa tgaagcccgt cgtctgcgtg ccgaacgctg gaaagcggaa 1920

aatcaggaag ggatggctga ggtcgcccgg tttattgaaa tgaacggatc gttcgccgac 1980

tgagacgagc tacctcatat ctagggagag aacgagacca tctgctcttg aatcactgaa 2040

gcttggaagg gcgaattcca gcacactggc ggccgttact aggatcc 2087

<210> 10
<211> 2087
<212> DNA
<213> Artificial Sequence

<220>
<223> DNA sequence of GDH-linker-Qred chimeric-ccdA construction
      reverse

<400> 10
atggcatata ccgacctgaa agataaagtt gttgttgtta ccggtggtag caaaggtctg 60

ggtcgtgcaa tggcagttcg ttttggtcaa gaacagagca aagttgtggt taattatcgc 120

agcaatgaag aagaagccct ggaagtcaaa aaagaaattg aacaggcagg cggtcaggca 180

attattgttc gtggtgatgt taccaaagaa gaggacgttg ttaatctggt tgaaaccgca 240
gttaaagaat ttggcaccct ggatgtgatg attaacaatg ccggtgttga aaatccggtt 300
ccgagccatg aactgagcct ggaaaattgg aatcaggtga ttgataccaa tctgaccggt 360
gcatttctgg gtagccgtga agccattaaa tactttgtgg aaaacgacat caaaggcaac 420
gtgattaata tgagcagcgt gcatgaaatg attccgtggc ctctgtttgt tcattatgca 480
gcaagcaaag gtggtatgaa actgatgacc gaaaccctgg cactggaata tgcaccgaaa 540
ggtattcgtg ttaataacat tggtccgggt gcaattgata ccccgattaa tgcagaaaaa 600
tttgcagatc cggaacagcg tgcagatgtt gaaagcatga ttccgatggg ttatattggt 660
aacccggaag aaattgcaag cgttgcagcc tttctggcaa gcagccaggc aagctatgtg 720
accggtatta ccctgtttgc agatggtggc atgaccaaat atccgagctt tcaggcaggt 780
cgtggtccga ccccgacacc gactcctacc cctacaccaa ccccgacccc gtcaagcccg 840
agtgatggtc cgtttccgaa agcaacaccg cagctgccga atagcgtttt tgatatgttt 900
agcatgaaag gcaaagtgac cgcaattaca ggtggtggtg gcggtattgg ttttgcagca 960
gcagaagcaa ttgccgaagc aggcggtgat gttgcactgc tgtatcgtag cgcaccgaat 1020
atggaagaac gtagcgcaga actggccaaa cgttttggtg ttaaagttaa aagctatcag 1080
tgcgaagtga ccgaacatga aagcgttaaa caggcaattg aagccgtgga aaaagatttt 1140
ggtcgtctgg attgttatat tgcaaatgcg ggtggcggtg ttccgggtag cattaacccg 1200
gattatccgc tggaagcatg gcataaaacc cagagcgtta atctgcatag caccttttat 1260
gcagcccgtg aatgtgcacg tatctttaaa gcacagggta gcggtagctt tattgcaacc 1320
accagtatta gcgcacgtat tgttaatgtt ccgtatgatc agcctgcata caatagcagc 1380
aaagcagcag ttgttcattt ttgtcgtagc ctggcacgtg attggcgtaa ttttgcacgt 1440
gtgaatacca tttcaccggg tttttttgat acaccgatgg gtccgagcga taaagcagtt 1500
gaagatgttc tgtatcagaa aagcgttctg ggacgtgccg gtgatgtgaa agaactgaaa 1560
gcagcatatc tgtatctggc cagcaatgca agcacctata ccacaggtgc cgatctgctg 1620
attgatggtg gttattgtct gacctaagga tcctagtaac ggccgccagt gtgctggaat 1680
tcgcccttcc aagcttcagt gattcaagag cagatggtct cgttctctcc ctagatatga 1740
ggtagctcgt ctcagtcggc gaacgatccg ttcatttcaa taaaccgggc gacctcagcc 1800
atcccttcct gattttccgc tttccagcgt tcggcacgca gacgacgggc ttcattctgc 1860
atggttgtgc ttaccagacc ggagatattg acatcatatg ccttgagcaa ctgatagctg 1920
tcgctgtcaa ctgtcactgt aatacgctgc ttcatgcctg cccctccctt ttggtgacca 1980
accggctcga cgggggcagc gcaaggcggt gcctccggcg ggccactcaa tgcttgagta 2040
tactcactag actttgcttc gcaaagtcgt gaccgcctac gggatcc 2087

<210> 11
<211> 1644
<212> DNA
<213> Artificial Sequence

<220>
<223> DNA sequence coding GDH-linker-Qred

<400> 11
atggcatata ccgacctgaa agataaagtt gttgttgtta ccggtggtag caaaggtctg 60

ggtcgtgcaa tggcagttcg ttttggtcaa gaacagagca aagttgtggt taattatcgc 120

agcaatgaag aagaagccct ggaagtcaaa aaagaaattg aacaggcagg cggtcaggca 180

attattgttc gtggtgatgt taccaaagaa gaggacgttg ttaatctggt tgaaaccgca 240

gttaaagaat ttggcaccct ggatgtgatg attaacaatg ccggtgttga aaatccggtt 300

ccgagccatg aactgagcct ggaaaattgg aatcaggtga ttgataccaa tctgaccggt 360

gcatttctgg gtagccgtga agccattaaa tactttgtgg aaaacgacat caaaggcaac 420

gtgattaata tgagcagcgt gcatgaaatg attccgtggc ctctgtttgt tcattatgca 480

gcaagcaaag gtggtatgaa actgatgacc gaaaccctgg cactggaata tgcaccgaaa 540

ggtattcgtg ttaataacat tggtccgggt gcaattgata ccccgattaa tgcagaaaaa 600

tttgcagatc cggaacagcg tgcagatgtt gaaagcatga ttccgatggg ttatattggt 660

aacccggaag aaattgcaag cgttgcagcc tttctggcaa gcagccaggc aagctatgtg 720

accggtatta ccctgtttgc agatggtggc atgaccaaat atccgagctt tcaggcaggt 780

cgtggtccga ccccgacacc gactcctacc cctacaccaa ccccgacccc gtcaagcccg 840

agtgatggtc cgtttccgaa agcaacaccg cagctgccga atagcgtttt tgatatgttt 900

agcatgaaag gcaaagtgac cgcaattaca ggtggtggtg gcggtattgg ttttgcagca 960

gcagaagcaa ttgccgaagc aggcggtgat gttgcactgc tgtatcgtag cgcaccgaat 1020

atggaagaac gtagcgcaga actggccaaa cgttttggtg ttaaagttaa aagctatcag 1080

tgcgaagtga ccgaacatga aagcgttaaa caggcaattg aagccgtgga aaaagatttt 1140

ggtcgtctgg attgttatat tgcaaatgcg ggtggcggtg ttccgggtag cattaacccg 1200

gattatccgc tggaagcatg gcataaaacc cagagcgtta atctgcatag caccttttat 1260

gcagcccgtg aatgtgcacg tatctttaaa gcacagggta gcggtagctt tattgcaacc 1320

accagtatta gcgcacgtat tgttaatgtt ccgtatgatc agcctgcata caatagcagc 1380

aaagcagcag ttgttcattt ttgtcgtagc ctggcacgtg attggcgtaa ttttgcacgt 1440

gtgaatacca tttcaccggg tttttttgat acaccgatgg gtccgagcga taaagcagtt 1500

gaagatgttc tgtatcagaa aagcgttctg ggacgtgccg gtgatgtgaa agaactgaaa 1560

gcagcatatc tgtatctggc cagcaatgca agcacctata ccacaggtgc cgatctgctg 1620

attgatggtg gttattgtct gacc 1644

<210>  12
<211>  5
<212>  PRT
<213>  Artificial

<220>
<223>  glycine-serine linker


<220>
<221>  MISC_FEATURE
<222>  (1)..(5)
<223>  fragment (sequence) from position 1 to 5 can be repeated n times,
       wherein n  is an integer from 2 to 5

<400>  12

Gly Gly Gly Gly Ser
1               5


<210>  13
<211>  7
<212>  PRT
<213>  Artificial

<220>
<223>  alfa-helix-forming peptide linker


<220>
<221>  HELIX
<222>  (2)..(6)
<223>  fragment (sequence) from position 2 to 6 can be repeated n times,
       wherein n
       is an integer from 2 to 5

<300>
<301>  Arai R et al.
<302>  Design of the linkers which effectiveky separate domains of a
       bifunctional fusion protein
<303>  Protein Engineering
<304>  14
<305>  8
<306>  529-532
<307>  2001-05-14

<400>  13

Ala Glu Ala Ala Ala Lys Ala
1               5